# EUROPÄISCHE PATENTSCHRIFT

(11) **EP 2 041 169 B1**
(45) Veröffentlichungstag und Bekanntmachung des Hinweises auf die Patenterteilung: **31.08.2011**
(21) Anmeldenummer: 07785902.3
(22) Anmeldetag: 05.07.2007
(51) Int. Cl.: C07K 14/62

(54) **AMIDIERTES INSULIN GLARGIN**
AMIDATED GLARGINE INSULIN
INSULINE GLARGINE AMIDIFIÉE

(30) Priorität: 11.07.2006 DE 102006031962
(43) Veröffentlichungstag der Anmeldung: 01.04.2009
(73) Patentinhaber: Sanofi-Aventis Deutschland GmbH, 65929 Frankfurt am Main (DE)
(72) Erfinder: HABERMANN, Paul, 65926 Frankfurt am Main (DE); ZOCHER, Frank, 65926 Frankfurt am Main (DE)
(86) Internationale Anmeldenummer: PCT/EP2007/005932
(87) Internationale Veröffentlichungsnummer: WO 2008/006496

(56) Entgegenhaltungen:
- EP-A2- 0 254 516
- WO-A-03/044210
- WO-A-03/105888
- WO-A-2006/015879
- US-A- 5 656 722
- SANGER F ET AL: "The amide groups of insulin." THE BIOCHEMICAL JOURNAL MAR 1955, Bd. 59, Nr. 3, März 1955 (1955-03), Seiten 509-518, XP002451290 ISSN: 0264-6021
- HARTMANN H ET AL: "Biological activity of des-(B26-B30)-insulinamide and related analogues in rat hepatocyte cultures." DIABETOLOGIA JUL 1989, Bd. 32, Nr. 7, Juli 1989 (1989-07), Seiten 416-420, XP002451291 ISSN: 0012-186X
- BARNETT A H ET AL: "Insulin analogues" LANCET THE, LANCET LIMITED. LONDON, GB, Bd. 349, Nr. 9044, 4. Januar 1997 (1997-01-04), Seiten 47-51, XP004843580 ISSN: 0140-6736
- LEYER S ET AL: "THE ROLE OF THE C-TERMINUS OF THE INSULIN B-CHAIN IN MODULATING STRUCTURAL AND FUNCTIONAL PROPERTIES OF THE HORMONE" INTERNATIONAL JOURNAL OF PEPTIDE AND PROTEIN RESEARCH, MUNKSGAARD, COPENHAGEN, DK, Bd. 46, Nr. 5, 1. November 1995 (1995-11-01), Seiten 397-407, XP000530836 ISSN: 0367-8377

## Beschreibung

Die Erfindung betrifft das durch Amidierung modifizierte Insulin Glargin Gly(A21), Arg(B31), Arg -Amid (B32) Humaninsulin (Insulin Glarginamid).

Weltweit leiden etwa 177 Mio. Menschen an Diabetes mellitus. Darunter sind etwa 17 Mio. Typ I-Diabetiker, für welche die Substitution der fehlenden endokrinen Insulinsekretion die einzige derzeit mögliche Therapie darstellt. Die Betroffenen sind lebenslang, in der Regel mehrmals täglich, auf Insulininjektionen angewiesen. Im Gegensatz zum Typ I-Diabetes besteht beim Typ II-Diabetes nicht grundsätzlich ein Mangel an Insulin, jedoch wird in einer Vielzahl von Fällen, vor allem im fortgeschrittenen Stadium, die Behandlung mit Insulin, gegebenenfalls in Kombination mit einem oralen Antidiabetikum, als günstigste Therapieform angesehen.

Beim Gesunden ist die Insulinfreisetzung durch die Beta- Zellen des Pankreas strikt an die Konzentration der Blutglucose gekoppelt. Erhöhte Blutglucosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten und die hepatische Glucoseproduktion in der Nacht niedrig zu halten. Der Ersatz der körpereigenen Insulinsekretion durch exogene, meist subkutane Applikation von Insulin erreicht in der Regel die oben beschriebene Qualität der physiologischen Regulation der Blutglucose nicht. Häufig kommt es zu Entgleisungen der Blutglucose, da Diabetiker die jeweilige Situation falsch einschätzen. Daneben stellen jedoch auch über Jahre erhöhte Blutglucosespiegel ohne anfängliche Symptome ein erhebliches Gesundheitsrisiko dar. Die groß angelegte sog. DCCT-Studie in den USA (The Diabetes Control and Complications Trial Research Group (1993) N. Engl. J. Med. 329, 977-986) wies eindeutig nach, dass chronisch erhöhte Blutglucosespiegel wesentlich für die Entwicklung diabetischer Spätschäden verantwortlich sind. Diabetische Spätschäden sind mikro- und makrovaskuläre Schädigungen, die sich u. U. als Retino -, Nephro -, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, dass eine verbesserte Therapie des Diabetes in erster Linie darauf abzielen muss, die Blutglucose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglucose auszugleichen. Langsam wirkende Basalinsuline sollen die Grundversorgung mit Insulin insbesondere während der Nacht sicherstellen, ohne zu einer Hypoglykämie zu führen.

Insulin ist ein Polypeptid aus 51 Aminosäuren, die sich auf 2 Aminosäureketten verteilen: die A Kette mit 21 Aminosäuren und die B-Kette mit 30 Aminosäuren. Die Ketten sind durch 2 Disulfidbrücken miteinander verbunden. Insulinzubereitungen werden seit vielen Jahren zur Diabetestherapie eingesetzt. Dabei werden nicht nur natürlich vorkommende Insuline verwendet, sondern neuerdings auch Insulinderivate und -analoga.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes mit anderen Aminosäureresten und/oder Addition/Entfernen wenigstens eines Aminosäurerestes von dem entsprechenden, ansonsten gleichen natürlich vorkommenden Insulin unterscheiden. US 5,656,722 z. B. beschreibt z.B. Insulin Glargin (Arg(B31), Arg (B32), Gly (A21) Humaninsulin). Es kann sich bei den hinzugefügten und / oder ersetzten Aminosäureresten auch um solche handeln, die nicht natürlich vorkommen.

Insulinderivate sind Derivate von natürlich vorkommenden Insulinen oder Insulinanaloga, bei denen einer oder mehrere Aminosäurereste und/oder die N- oder C-Termini der A- und/oder B-Kette durch funktionelle Gruppen substituiert sind. Die funktionellen Gruppen sind ausgewählt aus einer Gruppe enthaltend Amidreste, Aminreste, Carboxlyreste, Alkylreste, Alkoholreste und Alkoxyreste.

Eine effektive Insulintherapie macht Gebrauch von sogenannten Basal - Insulinen. Darunter versteht man Formulierungen, die ein langsames, kontinuierliches Freisetzen von exogen appliziertem Insulin ermöglichen. Dadurch wird über längere Zeit eine basale Insulinkonzentration im Körper erreicht, die sich vorteilhaft auf den physiologischen Zustand des an Diabetes erkrankten Menschen auswirkt. Idealerweise setzt die Insulinwirkung verzögert und als möglichst flaches Zeit / Wirkungsprofil ein, so dass die Gefahr einer kurzfristigen Unterzuckerung deutlich minimiert ist und die Applikation ohne vorherige Einnahme von Nahrungsmitteln erfolgen kann.

Das rekombinante Insulinanalog Arg(B31), Arg (B32), Gly (A21) Humaninsulin (Insulin Glargin; US 5,656,722) zeichnet sich dabei dadurch aus, dass es bei vielen Patienten nur alle 24 Stunden - also nur einmal am Tag - dem Körper zugeführt werden muss, um eine basale Wirkung zu erreichen. Es kommt zu einer verbesserten Blutzuckerkontrolle die z.B. zu einer Verringerung des Hba1c Wertes führt.

Es wurde nun überraschend gefunden, dass Insulin Glarginamid, welches durch Amidierung des Arginin in Position B32 von Insulin Glargin entsteht, ein deutlich flacheres und damit vorteilhafteres Zeit/ Wirkungsprofil zeigt als Insulin Glargin selbst. Bei Wirkungseintritt fällt der Blutzuckerwert ohne deutlichen Tiefstpunkt (Nadir) ab. Damit hat Insulin Glarginamid eine überraschend vorteilhafte Qualität im pharmakologischen Sinne. Die Gefahr einer Hypoglykämie bei der Applikation wird somit minimiert. Durch die Applikation von Lantusamid stellt sich ein normal nüchternen Blutzuckerspiegel von 70-110 mg/dl Glukose ein. Zudem zeigt Insulin Glarginamid gegenüber Insulin Glargin überraschend eine verlängerte Wirkung. Insulin Glarginamid ist somit Gegenstand der Erfindung.

Dem Fachmann ist dabei klar, dass das Insulin Glarginamid Gegenstand einer pharmazeutischen Formulierung ist. Darunter versteht man ein pharmazeutisches Gemisch, das sich nach Applikation vorteilhaft in bester Art und Weise die Wirkung von Insulin Glarginamid entfaltet. Dabei geht man von wässrigen Lösungen aus. Entsprechend müssen weitere Komponenten mischbar sein. Es ist vorteilhaft, dass die Zubereitung keine Komponenten enthalten sollte, die aus tierischen Quellen stammen. Damit wird die Gefahr viraler Kontamination minimiert. Es ist weiterhin vorteilhaft, durch Zusatz von Konservierungsmitteln eine mikrobielle Verunreinigung zu verhindern. Durch den Zusatz isotoner Agentien kann eine mögliche negative Auswirkung der Formulierung auf die Physiologie der Gewebezellen an der Applikationsstelle kompensiert werden. Stabilisierend kann sich der Zusatz von Protamin auswirken, so dass man weitgehend salzfreien Insulinzubereitung gelangen kann, wenn man der Formulierung Protamin zufügt. Der Zusatz von einer phenolischen Komponente kann zu einer Stabilisierung der Hexamerstruktur des Insulinanalogons führen und begünstigt so den Verzögerungseffekt beim Wirkungseintritt.

Das Glarginamid kann parallel zu schnellwirksamen Insulinen wie Apidra^{®}, NovoRapid^{®}, Humalog ^{®}oder sich in Entwicklung befindlichen Insulinderivaten oder Formulierungen mit entsprechendem Zeit - /Aktionsprofil gegeben werden. Dabei ist es dem Fachmann klar, dass dazu auch entsprechend formulierte Mischungen der jeweiligen Insulinkom-ponenten verwendet werden können. Das Amid kann zudem auch von Individuen bevorzugt genommen werden, die inhalierbares Insulin wie Exubera^{®} anwenden. Weiterhin kann Glarginamid in pharmazeutischen Zubereitungen verwendet werden, die Peptide, die durch eine dem GLP-1 (Glucagon like Peptide -1) vergleichbare Aktivität beschrieben sind, enthalten.

Beispiel für solche Peptide stellen GLP-1 (7-37), Exenatide ( Byetta^{®}) oder Peptide, deren Herstellung in den Patentanmeldungen WO 2006/058620, WO 2001/04156 und WO 2004/005342 beschreiben, dar.

Die am gleichen Tage wie diese Patentanmeldung eingereichte Patentanmeldung mit dem Titel "Verfahren zur Herstellung von Insulinanaloga mit dibasischem B-Kettenende" beschreibt u.a. ein Verfahren zur Herstellung von Insulin Glarginamid. Dabei wird über eine Trypsin-katalysierte Ligation Argininamid an Gly(A21), Arg (B31) Humaninsulin mit hoher Ausbeute addiert. Dabei kann man die Reaktion so steuern. dass bevorzugt das Insulinanaloga der Form Gly(A21), Arg(B31), Arg (B32) - Amid entsteht. In der Literatur wird beschrieben, dass insbesondere geschützte Argininderivate in verschiedenen Lösungsmitteln instabil sein können. Daher werden in der Peptidchemie stetig neue Schutzgruppen entwickelt, die eine verbesserte Stabilität bewirken. Entsprechend der Schutzgruppen oder Amidgruppe kann durch Variation der Reaktionsbedingungen die Ausbeute positiv beeinflusst werden kann. Dies ist dem Fachmann geläufig und auch Gegenstand der Erfindung. In dem Patent US 5,656,722 sind die Plasmide pB40 und pINT91d beschrieben, welche die Expression von Miniproinsulin als Teil eines Fusionsproteins erlauben. Wenn man Asparagin in Postion 21 der Insulin - A-Kette durch Glycin ersetzt, kann man aus diesen Fusionsproteinen direkt Arg(B31), Gly (A21) Miniproinsulin herstellen, das nach tryptischem Verdau direkt in die Vorstufe zur Herstellung von Insulin Glarginamid umgewandelt werden kann. Entsprechende Fusionsproteine müssen nicht notwendigerweise intrazellulär hergestellt werden. Es ist dem Fachmann klar, dass dieses Proinsulinanalogon auch durch bakterielle Expression mit anschließender Sekretion in das Periplasma und/oder in den Kulturüberstand hergestellt werden können. Die Europäische Patentanmeldung EP-A 1 364 029 beschreibt dies beispielhaft. Auch die Verwendung von Arg(B31), Gly (A21) - Humaninsulin - Vorstufen, die direkt nach Expression aus solchen bakteriellen Verfahren entstehen, ist Gegenstand der Erfindung.

Die Amidierung von Insulinanaloga kann generell an verschiedenen Stellen erfolgen. Insulin Glargin, bei welchem die C-terminale Carboxylgruppe amidiert ist, wird als Insulin Glarginamid bezeichnet.

Dem Fachmann ist ferner geläufig, dass die beispielhaft beschriebenen Expressionssysteme nur einen kleinen Ausschnitt der für die rekombinante Herstellung von Proteinen entwickelten WirtsNektorsysteme darstellen. Wirts/Vektor Systeme, die die Herstellung der Zielpeptide erlauben, sind somit auch Bestandteil der Erfindung.

Gegenstand der vorliegenden Erfindung ist somit amidiertes Insulin Glargin der Form Gly(A21), Arg (B31), Arg(B32)-NH₂ Humaninsulin (Insulin Glarginamid).

Ein weiterer Gegenstand der Erfindung ist ein Verfahren zur Herstellung eines Insulin Glarginamids, wobei ein Vorläufer des Insulin Glarginamids der Form Gly(A21), Arg(B31) Humaninsulin rekombinant hergestellt wird, eine Kupplung mit Argininamid in Gegenwart eines Enzyms mit Trypsinaktivität durchgeführt wird, und das Insulin Glarginamid isoliert wird.

Ein weiterer Gegenstand der Erfindung ist die Verwendung von Insulin Glarginamid in einem Verfahren zur Herstellung eines Medikaments zur Behandlung von Diabetes, insbesondere Diabetes Typ I oder Typ II Diabetes.

Ein weiterer Gegenstand der Erfindung ist ein Arzneimittel enthaltend Insulin Glarginamid, welches insbesondere eine wässrige Formulierung oder ein Pulver darstellt.

Das Arzneimittel ist eine pharmazeutische Zubereitung, die vorzugsweise eine Lösung oder Suspension zu Injektionszwecken ist; sie ist gekennzeichnet durch einen Gehalt an mindestens einem amidierten Insulin Glargin, insbesondere Insulinglarginamid, und/oder mindestens einem von deren physiologisch verträglichen Salzen in gelöster, amorpher und/oder kristalliner - vorzugsweise in gelöster -Form.

Die Zubereitung weist vorzugsweise einen pH-Wert zwischen etwa 2,5 und 8,5, insbesondere zwischen etwa 4,0 und 8,5 auf,
enthält ein geeignetes Isotoniemittel,
ein geeignetes Konservierungsmittel
und gegebenenfalls einen geeigneten Puffer,
sowie vorzugsweise auch eine bestimmte Zinkionen-Konzentration,
alles natürlich in steriler wässriger Lösung. Die Gesamtheit der
Zubereitungsbestandteile außer dem Wirkstoff bildet den Zubereitungs-Träger.

Geeignetes Isotoniemittel sind z.B. Glycerin, Glukose, Mannit, NaCl, Calcium- oder Magnesium-Verbindungen wie CaCl₂ etc.

Durch die Wahl des Isotoniemittels und/oder Konservierungsstoffes beeinflusst man die Löslichkeit des amidierten Insulin Glargins bzw. dessen physiologisch verträglichen Salzes bei den schwach sauren pH-Werten.

Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 4,0 und 8,5 können z.B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCI) bzw. Laugen (typischerweise NaOH) geeignet.

Wenn die Zubereitung einen Zinkgehalt besitzt, ist ein solcher von 1 µg/ml bis 2 mg/ml, insbesondere von 5 µg bis 200 µg Zink/ml bevorzugt.

Zwecks Variation des Wirkstoffprofils der erfindungsgemäßen Zubereitung kann auch unmodifiziertes Insulin, vorzugsweise Rinder-, Schweine- oder Human-Insulin, insbesondere Human-Insulin, oder Insulinanaloga und Derivate davon zugemischt werden. Ebenfalls können ein oder mehrere Exendin-4 Derivate oder Peptide, die durch eine dem GLP-1 (Glucagon like peptide-1) vergleichbare Aktivität charakterisiert sind, zugemischt werden. Solche Arzneimittel (Zubereitungen) sind ebenfalls Gegenstand der Erfindung.

Bevorzugte Wirkstoffkonzentrationen sind solche entsprechend etwa 1 - 1500, weiter bevorzugt etwa 5 - 1000 und insbesondere etwa 40 - 400 internationale Einheiten/ml.

### Figurenlegende

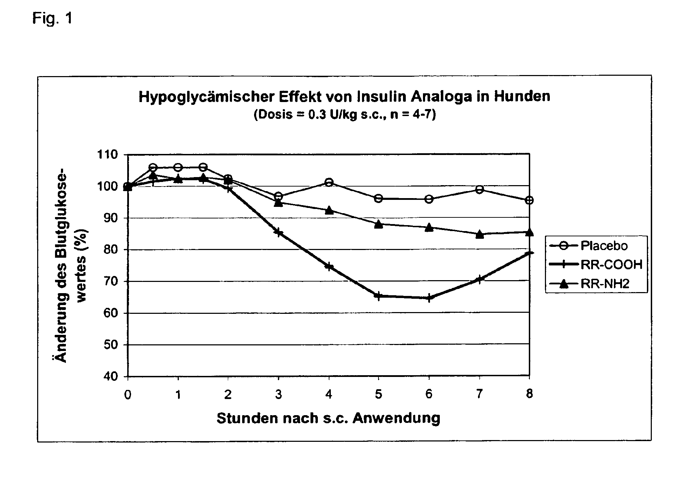

Fig. 1: Hypoglykämischer Effekt verschiedener Insulinanaloga in Hunden. RR-COOH = Insulin Glargin; RR-NH₂ = Insulin Glarginamid

Die Erfindung wird im Folgenden durch die Beispiele näher beschrieben, ohne sich auf diese zu beschränken.

### Beispiel 1: Gensequenz zu Sekretion eines Hirudin Arg(B31), Gly(A21) Insulin - Fusionsproteins durch Bäckerhefe

Die Patentanmeldung EP-A 1 364 032 schlägt die Verwendung von Hirudin als Fusionspartner zur Expression und Sekretion von pharmazeutisch interessanten Wertproteinen in Hefen vor.

Beispiel 1 der Patentanmeldung EP-A 1 364 032 beschreibt ein Wirtsvektorsystem zur Herstellung eines Fusionsproteins, das aus einem Hirudinderivat und Miniproinsulin besteht. Dieses System kann man für die Herstellung von Miniproinsulinen, die als Vorstufe zu dem beanspruchten Insulin Glarginamid führen, verwenden.

Die Konstruktion des Expressionsvektors erfolgt in Analogie zu dem Beispiel der

Patentanmeldung EP-A 1 364 032, indem man den Primer insnco1 rev ersetzt und so das Codon in Position A21 verändert wird.

Zur Herstellung der für ein Fusionsprotein enthaltend Miniproinsulin kodierenden Sequenz (wobei aus dem Miniproinsulin später Gly(A21), Arg(B31) Humaninsulin entsteht), wird folgender Primer synthetisiert:
ins_gly_a21_rev
TTTTTTCCATGGGTCGACTATCAGCCACAGTAGTTTTCCAGCTGG (SEQ ID NO.: 1)

Der Primer überdeckt dabei vollständig den für die Aminosäuren A15-A21 des Insulinanalogs kodierenden Genabschnitt. Kombiniert man diesen Primer mit dem Primer SEQ ID NO:4 aus Beispiel 1 der Patentanmeldung EP-A 1 364 032 und verwendet das Plasmid pADH2Hir_ins als 'Template', so lässt sich über PCR ein DNA Fragment generieren, dass nach Verdau mit den Restriktionsenzymen Kpnl und Ncol in den entsprechend geöffneten Expressionsvektor insertiert wird und das gewünschte Fusionsprotein enthält.

Der Vektor erhält die Bezeichnung pADH2Hir_ins_glyA21. Das Fusionsprotein wird entsprechend Beispiel 3 der der Patentanmeldung EP-A 1 364 032 exprimiert und entsprechend Beispiel 6 zu Gly(A21) - Miniproinsulin prozessiert und über Kationentausch-Chromatographie gereinigt. Das Material der Fraktion die Miniproinsulin enthält, wird zur Herstellung von Gly(A21), Arg(B31), Arg(B32)-NH₂ Humaninsulin entsprechend Beispiel 5 der vorliegenden Anmeldung verwendet.

### Beispiel 2: Gensequenz zur direkten Sekretion der Gly(A21), Arg(B31), Humaninsulin - Vorstufe durch Bäckerhefe

DNA des in Beispiel 7 der Patentanmeldung EP-A 1 364 032 beschriebenen Plasmides pADH2Hir_ins_glyA21 wird zur Herstellung eines Vektorkonstruktes zur direkten Sekretion von Gly(A21), Arg(B31) Humaninsulin verwendet.

Folgende Primer werden synthetisiert.
alpha_insf1
5'- TTTTTTGGATCCTTTGGAATAAAAGATTTGTTAACCAACACTTGTGTG-3' (SEQ ID NO.: 2)

Er überdeckt die Sequenz kodierend den C-Terminus des alpha - Faktors, Codone für Lys-Arg und des N-Terminus der Miniproinsulin Sequenz.
ins_gly_rev2
5' - TTTTTTCCAT GGGTCGCTAT CAGCCACAGT AGTTTTCCAG CTGG -3' (SEQ ID NO.: 3)
Der Primer hybridisiert mit dem 3'Ende der in das Plasmid pADH2Hir_ins_glyA21 klonierten Insulinanaloga - Sequenz. In einer PCR (Standardbedingungen) wird ein DNA Fragment generiert, das nach Verdau mit den Restriktionsenzymen Kpnl und Ncol in den entsprechend geöffneten Expressionsvektor insertiert wird und das gewünschte Fusionsprotein enthält. Das in kompetente Zellen des Hefestammes Y79 transformiert. Transformanten werden anschließend wie in Beispiel 7 der genannten Patentanmeldung beschrieben, exprimiert

Gly(A21), Arg(B31) - Miniproinsulin wird entsprechend EP-A 0 347 781, Beispiel 9, angereichert und nach Trypsinspaltung über Kationenaustausch - Chromatographie gereinigt. Material der Fraktion, die Miniproinsulin enthält, wird zur Herstellung von Gly(A21), Arg(B31), Arg(B32) Humaninsulin entsprechend Beispiel 5 der vorliegenden Anmeldung verwendet.

### Beispiel 3: Gensequenz zu Sekretion eines Hirudin - Gly(A21), Arg(B31) Humaninsulin -Fusionsproteins durch Pichia pastoris

Die Klonierung des Expressionsvektors erfolgt in Analogie zu Beispiel 4 der Patentanmeldung EP-A 1 364 032. Anstelle der Sequenz Primer pichia_H_ Irev2 wird dabei der Primer ins_gly_rev2, der später die Möglichkeit der Expression von Gly(A21) Humaninsulin mit dem PCR-Produkt ermöglicht, eingesetzt :
5'- TTTTTGGCGCCGAATTCACTACTATTAGCCACAGTAGTTTTCCAGCTGG - 3' (SEQ ID NO.:4)

Das entstandene Plasmid erhält die Bezeichnung pPich_Hir_ins-GlyA21. Die Reinigung von Gly(A21), Arg(B31) - Miniproinsulin als Ausgangsmaterial wird wie beschrieben durchgeführt.

### Beispiel 4: Gensequenz zur direkten Sekretion der Gly(A21), Arg(B31) - Vorstufe durch Pichia pastoris

In Analogie zu Beispiel 7 der Patentanmeldung EP-A 1 364 032 erfolgt die Konstruktion des entsprechenden Expressionsvektors. Man benötigt die DNA des Plasmides pPich_Hir-ins-GlyA21 und zwei Primer pich_insgly_dirf und pich_insgly_dirrev
pich_insgly_dirf
5'-TTTTTTCTCGAGAAAAGATTTGTTAACCAACACTTGTGTG-3' (SEQ ID NO.: 5)
pich_insgly_dirrev
5'-TTTTTT GGCGCCGAATTCACTACTATTAGCCAC-3' (SEQ ID NO.: 6)

Die Reinigung von Gly(A21), Arg(B31) - Miniproinsulin als Ausgangsmaterial wird wie beschrieben durchgeführt.

### Beispiel 5: Herstellung von Gly(A21), Arg(B31), Arg(B32) - NH₂ - Humaninsulin aus einer Gly(A21), Arg(B31) Humaninsulin - Vorstufe über Kupplung mit Argininamid

100 mg Gly(A21), Arg(B31) Humaninsulin, die entsprechend den Beispielen 1-4 dargestellt wurden, werden in 0.95 ml Argininamidlösung (446 g/L) gelöst, 0.13 mL M Na-Acetatpuffer (pH 5.8) und 2 ml DMF zugegeben. Die Reaktionsmischung wird auf 12° C gekühlt und durch Zugabe von 0.094 ml Trypsin (0.075 mg, Roche Diagnostics) gestartet.

Nach 8 h wird die Reaktion durch Zugabe von TFA bis pH 2.5 gestoppt und per HPLC analysiert. Es bildet sich >60 % Gly(A21),Arg(B31),Arg(B32) Humaninsulin. Nach Zusatz von Trypsininhibitorlösung erfolgt die Reinigung des amidierten Analogons (Insulin Glarginamid) in Analogie zu US 5,656,722.

### Beispiel 6: Gensequenz zur direkten Sekretion einer Lys(B31) - Vorstufe durch Bäckerhefe

Zwei Primer werden synthetisiert, die zur Einfüfrung der Sequenz Ala(B30), Ala(C1), Lys(C2) dienen:
a29_a30_k31f
5'-GTTTCTTCTACACTCCAAAGGCGGCTAAAGGTATCGTTGAACAATGTTG-3' (SEQ ID NO.: 7)
und a29_a30_k31rev
5'-CAACATTGTT CAACGATACC TTTAGCCGCC TTTGGAGTGT AGAAGAAAC -3' (SEQ ID NO.: 8)

Der Primer alpha_insf1
5'- TTTTTTGGATCCTTTGGAATAAAAGATTTGTTAACCAACACTTGTGTG-3' (SEQ ID NO.: 9)
überdeckt die Sequenz des C-Terminus des alpha - Faktors, Codone für Lys-Argund des N-Terminus der Miniproinsulin Sequenz. DNA des Plasmides pADH2Hir_ins aus Beispiel 1 der Anmeldung WO 02/070722A1 dient als Template für zwei Polymerasekettenrektionen. In Reaktion 1 werden die Primer a29_a30_k31f und
insnco1 rev (SEQ ID NO.:6 aus WO 02/070722A1) und in Reaktion 2 die Primer a29_a30_k31 re und alpha_insf1 benutzt. Die Standardreaktionen werden durchgeführt und die entstandenen PCR - Fragmente isoliert. Aliquots der beiden Ausbeuten werden vereinigt und dienen als Template für eine dritte Reaktion mit den Primern insnco1 rev und SEQ ID NO:6 aus WO 02/070722A1. Das entstandene PCR - Fragment wird wie in Beispiel 8 beschrieben kloniert und exprimiert. Es entsteht Ala (B31), Ala(C1), Lys(C1) -Miniproinsulin, das mit Lysylendopeptidase in B(1-29) - A(1-21) Split Insulin umgewandelt wird und als Intermediat zur Herstellung des Zielproteins dient.

### Beispiel 7: Umsetzung mit Lysylendopeptidase und Thr-Arg(Boc)-Arg(Boc)-NH2

Die Insulinvorstufe wird wie in DE3844211 beschrieben mit Lysylendopeptidase (LEP) und Trypsin umgesetzt und gereinigt (Beispiel 1). Hierzu werden 10 mg der Insulinvorstufe in Tris-Puffer (pH 8.0) gelöst und LEP aus Lysobacter enzymogenes zugegeben (0.01 ml einer 1 mg/ml konz. Lösung in Wasser, Merckbiosciences). Es wird bei Raumtemperatur 2 h inkubiert und über RP-HPLC aufgereinigt (Nucleosil 120-5 Säule). Die Gly (A21) B(1-29) - A(1-20) Split Insulinvorstufe wird in Anlehnung an DE3844211 mit Thr-Arg(Boc)-Arg(Boc)-NH2 umgesetzt. Hierzu werden 10 mg der desThr-Insulinvorstufe in 0.25 ml 10 M Essigsäure gelöst und 0.7 ml 1.5 M Thr-Arg(Boc)-Arg(Boc)-NH2 in DMSO/1,3-Butandiol (1:1) gelöst zugegeben. Es wird dann 0.15 ml LEP (15 mg/ml, gelöst in Wasser). Es wird bei Raumtemperatur 2 h inkubiert. Das Protein wird dann durch Zugabe von 5 ml Methanol/Methyl-tert.-butylether (v/v = 1:4) gefällt und getrocknet. Die Abspaltung der Schutzgruppe erfolgt durch Zugabe von 1 M HCl/Essigsäure und Inkubation bei 0°C.

### Beispiel 8: Blutzuckersenkende Wirkung von Insulin Glarginamid

Der blutzuckersenkende Effekt wird an gesunden männlichen Hunden der Rasse Beagle untersucht. Subkutan wird eine Dosis von 0,3 IU/kg Körpergewicht verabreicht. In einer Kontrollgruppe werden Hunde mit der gleichen Dosis Insulin Glargin behandelt und eine weitere Gruppe erhält eine Placeboinjektion ohne Insulinzusatz. Während der ersten 2 Stunden nach Injektion wird den Tieren jede halbe Stunde zur Blutzuckerbestimmung Blut entnommen, danach bis zur achten Stunde stündlich. Es zeigt sich, dass Insulin Glarginamid wie auch Insulin Glargin ein verzögertes Zeit/Wirkungsprofil aufweisen, jedoch ist das Profil des Amids deutlich vorteilhaft, da bei Wirkungseintritt der Blutzuckerwert ohne deutlichen Tiefpunkt (Nadir) abfällt. Das Ergebnis ist in Figur 1 dargestellt.

## Patentansprüche

1. Gly(A21), Arg (B31), Arg(B32)-NH₂ Humaninsulin (Insulin Glarginamid).

2. Verfahren zur Herstellung von Insulin Glarginamid gemäß Anspruch 1.

3. Verfahren gemäß Anspruch 2 zur Herstellung von Insulin Glarginamid gemäß Anspruch 1, wobei ein Vorläufer des Insulin Glarginamids der Form Gly(A21), Arg(B31) Humaninsulin rekombinant hergestellt wird, eine Kupplung mit Argininamid in Gegenwart eines Enzyms mit Trypsinaktivität durchgeführt wird, und das Insulin Glarginamid isoliert wird.

4. Verwendung von Insulin Glargin amid gemäß Anspruch 1 in einem Verfahren zur Herstellung eines Medikaments zur Behandlung von Diabetes.

5. Verwendung gemäß Anspruch 4, wobei Insulin Glarginamid in einem Verfahren zur Herstellung eines Medikaments zur Behandlung von Diabetes Typ I oder Typ II verwendet wird.

6. Arzneimittel enthaltend Insulin Glarginamid gemäß Anspruch 1.

7. Arzneimittel gemäß Anspruch 6, welches eine wässrige Formulierung darstellt.

8. Arzneimittel gemäß Anspruch 6, welches ein Pulver darstellt.

9. Arzneimittel gemäß Anspruch 7, in dem das Insulin Glarginamid in gelöster, kristalliner und/oder amorpher Form vorhanden ist.

10. Arzneimittel gemäß Anspruch 7, der zusätzlich ein oder mehrere Exendin-4 Derivate oder Peptide, die durch eine dem GLP-1 (Glucagon like peptide-1) vergleichbare Aktivität charakterisiert sind, enthält.

## Claims

1. Gly(A21), Arg (B31), Arg(B32)-NH₂ human insulin (insulin glargine amide).

2. A method for preparing insulin glargine amide as claimed claim 1.

3. The method as claimed in claim 2 for preparing insulin glargine amide as claimed in claim 1, where a precursor of insulin glargine amide of the form Gly(A21), Arg(B31) human insulin is prepared recombinantly, a coupling is carried out with argininamide in the presence of an enzyme having trypsin activity, and the insulin glargine amide is isolated.

4. The use of insulin glargine amide as claimed in claim 1 in a method for manufacturing a medicament for the treatment of diabetes.

5. The use as claimed in claim 4, where insulin glargine amide is used in a method for manufacturing a medicament for the treatment of diabetes of type I or type II.

6. A pharmaceutical comprising insulin glargine amide as claimed in claim 1.

7. A pharmaceutical as claimed in claim 6, which represents an aqueous formulation.

8. A pharmaceutical as claimed in claim 6, which represents a powder.

9. A pharmaceutical as claimed in claim 7, in which the insulin glargine amide is present in dissolved, crystalline and/or amorphous form.

10. A pharmaceutical as claimed in claim 7, which additionally comprises one or more exendin-4 derivatives or peptides which are **characterized by** an activity comparable to that of GLP-1 (glucagon like peptide-1).

## Revendications

1. Insuline humaine Gly(A21), Arg(B31), Arg(B32)-NH2 (insuline glarginamide).

2. Procédé de préparation d'insuline glarginamide selon la revendication 1.

3. Procédé selon la revendication 2, pour la préparation d'insuline glarginamide selon la revendication 1, dans lequel on prépare par recombinaison un précurseur de l'insuline glarginamide se présentant sous la forme insuline humaine Gly(A21), Arg (B31), on procède à un couplage avec de l'argininamide en présence d'une enzyme ayant une activité de trypsine, et on isole l'insuline glarginamide.

4. Utilisation de l'insuline glarginamide selon la revendication 1 dans un procédé de fabrication d'un médicament pour le traitement du diabète.

5. Procédé selon la revendication 4, dans lequel on utilise l'insuline glarginamide dans un procédé de fabrication d'un médicament pour le traitement du diabète de type I ou de type II.

6. Médicament contenant de l'insuline glarginamide selon la revendication 1.

7. Médicament selon la revendication 6, qui se présente sous forme d'une formulation aqueuse.

8. Médicament selon la revendication 6, qui se présente sous forme d'une poudre.

9. Médicament selon la revendication 7, dans lequel l'insuline glarginamide est présente sous une forme dissoute, cristalline et/ou amorphe.

10. Médicament selon la revendication 7, qui contient en outre un ou plusieurs dérivés de l'exendine-4 ou peptides exendine-4, qui sont **caractérisés par** une activité comparable à celle du GLP-1 (Glucagon like peptide-1).


### SEQUENCE LISTING

<110> Sanofi-Aventis Deutschland GmbH
<120> Amidiertes Insulin Glargin
<130> DE2006/026
<140> 10 2006 031 962.1 <141> 2006-07-11
<160> 9
<170> PatentIn version 3.3
<210> 1
   <211> 45
   <212> DNA
   <213> Artificial
<220>
   <223> Primer ins_gly_a21_rev
<400> 1
   ttttttccat gggtcgacta tcagccacag tagttttcca gctgg 45
<210> 2
   <211> 48
   <212> DNA
   <213> Artificial
<220>
   <223> Primer alpha_insf1
<400> 2
   ttttttggat cctttggaat aaaagatttg ttaaccaaca cttgtgtg 48
<210> 3
   <211> 44
   <212> DNA
   <213> Artificial
<220>
   <223> Primer ins_gly_rev2
<400> 3
   ttttttccat gggtcgctat cagccacagt agttttccag ctgg 44
<210> 4
   <211> 49
   <212> DNA
   <213> Artificial
<220>
   <223> Primer ins_gly_rev2
<400> 4
   tttttggcgc cgaattcact actattagcc acagtagttt tccagctgg 49
<210> 5
   <211> 40
   <212> DNA
   <213> Artificial
<220>
   <223> Primer pich_insgly_dirf
<400> 5
   ttttttctcg agaaaagatt tgttaaccaa cacttgtgtg 40
<210> 6
   <211> 33
   <212> DNA
   <213> Artificial
<220>
   <223> Primer pich_insgly_dirrev
<400> 6
   ttttttggcg ccgaattcac tactattagc cac 33
<210> 7
   <211> 49
   <212> DNA
   <213> Artificial
<220>
   <223> Primer a29_a30_k31f
<400> 7
   gtttcttcta cactccaaag gcggctaaag gtatcgttga acaatgttg 49
<210> 8
   <211> 49
   <212> DNA
   <213> Artificial
<220>
   <223> Primer a29_a30_k31rev
<400> 8
   caacattgtt caacgatacc tttagccgcc tttggagtgt agaagaaac 49
<210> 9
   <211> 48
   <212> DNA
   <213> Artificial
<220>
   <223> Primer alpha_insf1
<400> 9
   ttttttggat cctttggaat aaaagatttg ttaaccaaca cttgtgtg 48